# EUROPEAN PATENT APPLICATION

(11) **EP 0 691 139 A1**
(43) Date of publication of application: **10.01.1996**
(21) Application number: 95201430.6
(22) Date of filing: 01.06.1995
(51) Int. Cl.: A61M 39/28

(54) **Elastic clip for closing small flexible tubes**

(30) Priority: 07.07.1994 IT RE940039 U
(71) Applicant: C.G.M. S.P.A., I-42015 Corregio (Reggio Emilia) (IT)
(72) Inventor: Parmigiani, Corrado Senior, I-42015 Correggio (Reggio Emilia) (IT)
(74) Representative: Corradini, Corrado

(57) **Abstract**

The clip comprises an upper arm (11) and a lower arm (12) joined together by an elastically flexible strip (10), and hooking means (15, 19, 21) for mutually retaining said arms (11, 12); two members (30, 40) are provided projecting from said arms (11, 12) towards the interior central region of the clip to squeeze a tube (2) interposed between them and hence close its passageways when the arms (11, 12) are at their minimum distance apart; one projecting member (40) defines for the tube (2) a concave support surface (42) which is relatively wide and is of cylindrical shape with its directrix in the form of a circular arc; the other projecting member (30) has a relatively thin pressing edge (31) which faces the support surface (42) to act on the tube (2) and has a convex arcuate profile; the distance between the pressing edge (31) and the support surface (42) is substantially constant when the arms (11, 12) are at their minimum distance apart.

## Description

This invention relates to elastic clips for closing small flexible tubes, in particular for medical use, ie tubes for the passage of liquids from or into a patient's body.

Clips are known formed in one piece from plastics material, having an upper arm and a lower arm joined together by an elastically flexible arcuate strip which tends to maintain said arms spaced apart.

The two arms also possess projections directed towards the interior of the clip to flatteningly squeeze, along a rectilinear squeezing line, a tube interposed between them to close its passageway when said arms are brought to their minimum distance apart by the action of the fingers.

The clip also possesses hooking means which mutually retain the arms when at their minimum distance apart.

An object of the present invention is to improve the tube squeezing action so as to require lesser force exerted by the fingers and to achieve more effective closure of the passageway.

A further object is to maintain the clip in a geometrically correct position relative to the tube, ie arranged such that the tube cross-section which is squeezed is exactly perpendicular to the longitudinal axis of the tube; this is to improve the squeezing and closure action on the tube.

This and further objects are attained by the present invention as characterised in the claims.

The invention is described in detail hereinafter with the aid of the accompanying figures, which illustrate one embodiment thereof.
Figure 1 is a side view, to an enlarged scale, of the clip in its "open" configuration.
Figure 2 shows the view of Figure 1 but in the "closed" configuration.
Figure 3 is a section on the plane III-III of Figure 1.

The clip is formed in one piece from synthetic resin and comprises an upper arm 11 and a lower arm 12 joined together by an elastically flexible arcuate strip 10 which tends to maintain said arms spaced apart.

The terms "upper" and "lower" used herein for greater descriptive clarity are to be understood in the relative sense with reference to the accompanying figures, it being evident that the clip can assume any spatial orientation.

Specifically, the arms 11 and 12 and the strip 10 define a single substantially flat U-bent element the branches of which are defined by the arms 11 and 12.

By simultaneously pressing with the fingers on said arms 11 and 12, these approach each other with bending of the strip 10.

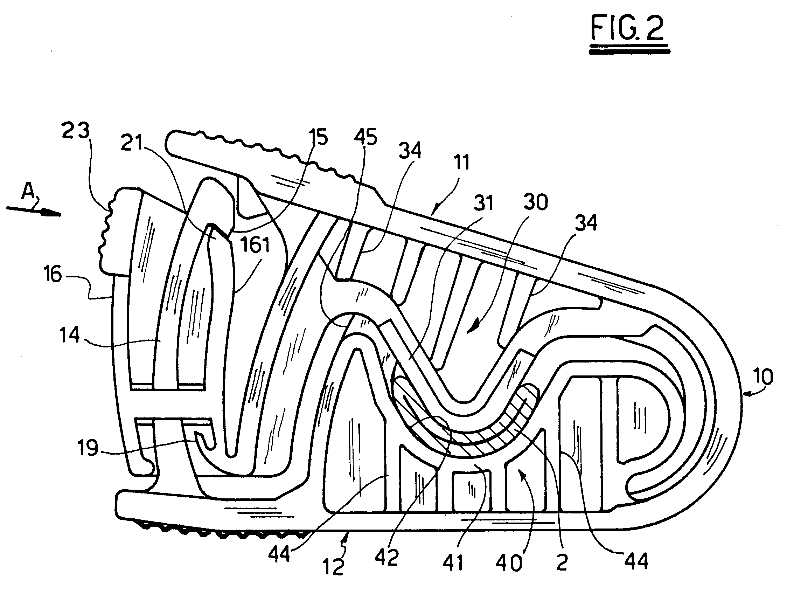

Hooking means are fixed to the front portions of the arms 11 and 12 to mutually retain these arms when they are positioned at their minimum distance apart ("closed" position - Figure 2).

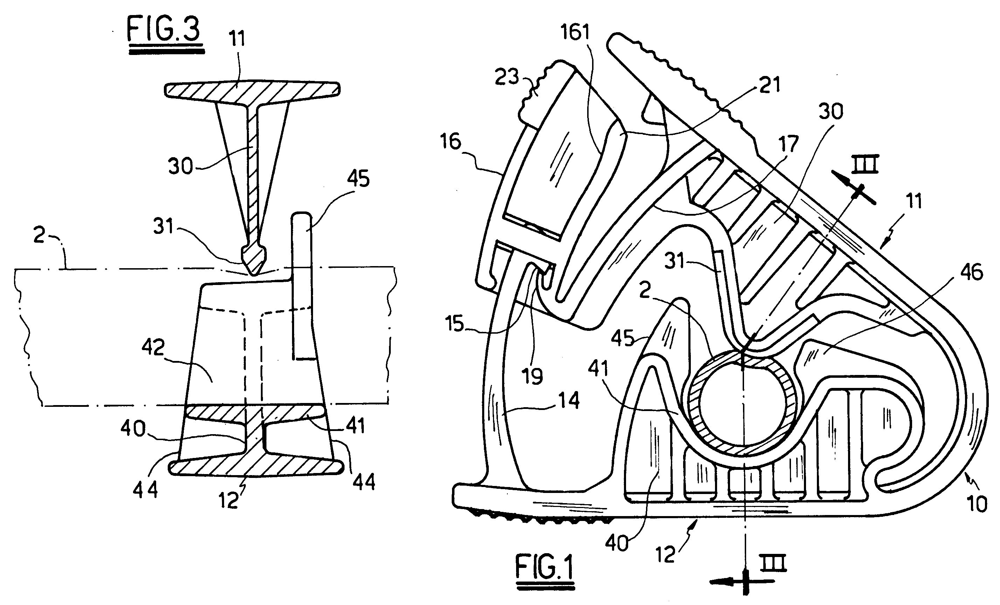

Said hooking means are also arranged to mutually retain the arms 11 and 12 when in their "open" position in which their distance apart is greater (Figure 1), as hereinafter specified.

In particular, the hooking means illustrated by way of example comprise a thin vertical element 14 extending upwards from the front end of the lower arm 12 and carrying at its upper end a tooth 15; the element 14 slides through a hollow body 16 having roughly the shape of a channel of rectangular cross-section with its cavity substantially vertical.

The body 16 is fixed to the end of a strip 17 which projects downwards from the upper arm 11.

The inner wall 161 of the body 16 carries at its lower end an outwardly projecting tooth 19, whereas its upper end defines a second tooth 21. The two teeth 19 and 21 are arranged to engage the tooth 15.

When the clip is in its "open" position, the tooth 15 engages the tooth 19 and the arms 11 and 12 cannot be moved further apart (Figure 1).

When the arms 11 and 12 are pushed one towards the other the tooth 15 finally engages the tooth 21 to hence define the "closed" position (Figure 2). To release the clip from this position, a finger is pressed inwardly (arrow A in Figure 2) against a knurled region 23 of the body 16. The body 16 moves elastically inwards to hence disengage the tooth 15 from the tooth 21, the strip 10 then returning the arms 11 and 12 to their "open" position.

To the arms 11 and 12, in a central position thereof, there are joined two members 30 and 40 respectively, which project towards the interior central region of the clip to squeeze a flexible tube interposed between them and close its passageway when the arms 11 and 12 are at their minimum distance apart ("closed" position).

The lower projecting member 40 has an upper edge 41 defining a concave surface 42 on which the tube rests. The surface 42 is of cylindrical shape with its directrix in the form of a circular arc, and is relatively wide, ie the length of its generators is virtually equal to the width of the arms 11 and 12.

The upper projecting member 30 is in the form of a thin plate, its lower edge 31, which presses against the tube 2, being relatively thin and in the shape of a rounded point in cross-section (Figure 3).

The edge 31 faces the support surface 42 and is of convex arcuate profile. Moreover the distance between any point on the edge 31 and the surface 42 is substantially constant when the arms 11 and 12 are in their "closed" position. This distance is such as to determine closure of the passageway through the tube 2.

Preferably, the curvature of the support surface 42 is such as to substantially match the curvature of the tube 2 when this is undeformed.

When the arms 11 and 12 are in their "open" position the edge 31 slightly interferes with the diameter of the tube positioned on the surface 42. This causes the clip to remain securely retained on the tube 2 even when in its "open" position.

Because of the shape of the support surface, which embraces the tube 2 through nearly 180°, the clip and the tube 2 securely remain in the required correct mutual position, ie one transverse to the other at 90°.

When the arms 11 and 12 are pressed together into their "closed" position, the tube 2 is squeezed between the edge 31 and the surface 42 to assume a U-shaped arcuate squashed form and the passageway through the tube is closed.

During this pressing together, the edge 31 acts as a sort of wedge against the tube 2, especially with regard to the reaction against the squeezing deriving from the ends of the U-squashed cross-section (the reaction exerted by the tube is greatest at these ends, where the two parts of the cross-section which are squeezed join together); hence the reaction exerted by these critical regions of the tube provides less effective opposition to the mutual approach of the arms 11 and 12, with the result that a smaller force is required from the fingers to achieve this approach, as is evidently preferable.

In the illustrated embodiment, virtually vertical ribs 34 and 44 are provided to strengthen the member 30 and member 40 respectively.

Shaped fins 45 and 46 are also provided which project upwards from the upper edge 41 to the side of the member 31 when the arms 11 and 12 are forced together. These fins serve to maintain the two arms 11 and 12 coplanar when the upper arm moves towards the lower arm, in particular when the force required for squeezing the tube is relatively large.

Modifications of a practical and applicational nature can be applied to the invention without leaving the inventive idea as hereinafter claimed.

## Claims

1. For closing small flexible tubes, an elastic clip formed in one piece from synthetic resin, comprising:
an upper arm (11) and a lower arm (12) joined together by an elastically flexible strip (10) which tends to maintain the arms (11, 12) spaced apart;
hooking means (15, 19, 21) for mutually retaining said arms (11, 12) when positioned at their minimum distance apart; and
two members (30, 40) projecting from said arms (11, 12) towards the interior central region of the clip to squeeze a tube (2) interposed between them and hence close its passageways when the arms (11, 12) are at their minimum distance apart;
characterised in that:
one said projecting member (40) defines a concave support surface (42) for the tube (2), said surface (42) being relatively wide and of cylindrical shape with its directrix in the form of a circular arc;
the other projecting member (30) has, acting on the tube (2), a relatively thin pressing edge (31) which faces the support surface (42) and has a convex arcuate profile;
the distance between the pressing edge (31) and the support surface (42) being substantially constant when the arms (11, 12) are at their minimum distance apart.

2. A clip as claimed in claim 1, characterised in that the curvature of the support surface (42) is such as to substantially match the curvature of the undeformed tube (2).
